# EUROPEAN PATENT APPLICATION

(11) **EP 4 378 409 A1**
(43) Date of publication of application: **05.06.2024**
(21) Application number: 22848715.3
(22) Date of filing: 01.08.2022
(51) Int. Cl.: A61B 18/12

(54) **HIGH-VOLTAGE TRANSMITTING CIRCUIT FOR CATHETER, AND ABLATION TOOL**

(30) Priority: 30.07.2021 CN 202110872792
(71) Applicant: Accupulse Medical Technology (Suzhou) Co., Ltd., Jiangsu 215127 (CN)
(72) Inventor: ZHAO, Chenggang, Suzhou, Jiangsu 215127 (CN); YUE, Youfu, Suzhou, Jiangsu 215127 (CN); ZHANG, Xiangming, Suzhou, Jiangsu 215127 (CN)
(74) Representative: Lavoix
(86) International application number: PCT/CN2022/109415
(87) International publication number: WO 2023/006111

(57) **Abstract**

A high-voltage transmitting circuit (100) for a catheter, and an ablation tool (1000). A first electrode (P1) and a second electrode (P2) are provided on the catheter. The high-voltage transmitting circuit (100) comprises: a first transmitting circuit (110), wherein a first end of the first transmitting circuit (110) is connected to a high-voltage positive electrode (+HV), a second end of the first transmitting circuit (110) is connected to a high-voltage ground (GND), and a third end of the first transmitting circuit (110) is connected to a high-voltage negative electrode (-HV); a filtering circuit (120), wherein an input terminal of the filtering circuit (120) is connected to a transmitting terminal of the first transmitting circuit (110), an output terminal of the filtering circuit (120) is connected to the first electrode (P1), and the second electrode (P2) is connected to the high-voltage ground (GND); and a control circuit (130), wherein the control circuit (130) is used for controlling the first transmitting circuit (110) to output an alternating-current (AC) square wave, which is subjected to filter processing by the filtering circuit (120) to form a sine wave between the first electrode (P1) and the second electrode (P2). In this way, the output of pulsed voltage waveform of the sine wave is realized, and therapeutic requirements in some ablation operations by pulsed electric field are satisfied, thereby expanding the application prospects of ablation technique by pulsed electric field in arrhythmia treatment.

## Description

### TECHNICAL FIELD

The present application relates to the technical field of pulsed electric field ablation, and in particular to a high-voltage transmitting circuit for a catheter and an ablation tool.

### BACKGROUND ART

Generally, the ablation energy in the catheter ablation technology currently used to treat arrhythmias is mainly based on radiofrequency energy, supplemented by cryoenergy. These two ablation methods have shown certain advantages in the treatment of arrhythmias, but they also have corresponding limitations. For example, ablation energy lacks selectivity in destroying tissues in the ablation area, and relies on the adhesion force of the catheter, which may cause certain damage to the adjacent esophagus, coronary arteries, and phrenic nerves. Therefore, relevant technologies to find a fast, safe, and efficient ablation energy to complete and achieve durable pulmonary vein isolation without damaging adjacent tissues have become a research focus.

Pulsed electric field ablation technology is a new ablation method that uses pulsed electric field as energy. As a non-thermal ablation technology, it has increasingly attracted attention in clinical applications. Pulsed electric field ablation technology mainly generates a high-voltage pulsed electric field with a pulse width of milliseconds, microseconds or even nanoseconds to release extremely high energy in a short period of time, so that a large number of irreversible micropores will be generated on cell membranes and even intracellular organelles such as the endoplasmic reticulum, mitochondria, cell nuclei, etc., which will cause the apoptosis of diseased cells, thereby achieving the expected therapeutic purpose. However, as a new energy ablation technology, pulsed electric field ablation technology has the defect that sine wave voltage output cannot be achieved between the transmitting electrodes, thereby limiting the clinical application of pulsed electric field ablation technology.

### CONTENTS OF THE APPLICATION

The present application aims to solve one of the technical problems in the related technology, at least to a certain extent. To this end, the first object of the present application is to provide a high-voltage transmitting circuit for a catheter, which realizes the output of pulsed voltage waveform of a sine wave, and meets the needs of treatment in some pulsed electric field ablation surgeries, thereby improving the application prospects of pulsed electric field ablation technology in the treatment of arrhythmia.

A second object of the application is to provide an ablation tool.

In order to achieve the above objects, in a first aspect, the present application provides a high-voltage transmitting circuit for a catheter, wherein a first electrode and a second electrode are provided on the catheter, and the high-voltage transmitting circuit comprises: a first transmitting circuit, wherein a first end of the first transmitting circuit is connected to the high-voltage positive electrode, a second end of the first transmitting circuit is connected to the high-voltage ground, and a third end of the first transmitting circuit is connected to the high-voltage negative electrode; a filtering circuit, wherein an input terminal of the filtering circuit is connected to a transmitting terminal of the first transmit circuit, an output terminal of the filtering circuit is connected to the first electrode, and the second electrode is connected to the high-voltage ground; a control circuit, wherein the control circuit is connected to the first transmitting circuit, and is used to control the first transmitting circuit so that the first transmitting circuit outputs an alternating-current (AC) square wave, which is subjected to filter processing by the filtering circuit to form a sine wave between the first electrode and the second electrode.

According to the high-voltage transmitting circuit for a catheter according to an embodiment of the present application, the first transmitting circuit is connected to the filtering circuit and the control circuit respectively, wherein the control circuit is used to control the first transmitting circuit so that the first transmitting circuit outputs an AC square wave, and a filtering process is performed by a filtering circuit to form a sine wave between the first electrode and the second electrode. As a result, the output of pulsed voltage waveform of a sine wave is realized, which meets the treatment needs in some pulsed electric field ablation surgeries, thereby improving the application prospects of pulsed electric field ablation technology in the treatment of arrhythmia.

According to an embodiment of the present application, the first transmitting circuit comprises: a first switch tube, a second switch tube and a third switch tube; wherein a first end of the first switch tube is connected to the high-voltage positive electrode; a second end of the first switch tube, a first end of the second switch tube, and a first end of the third switch tube are all connected to an input terminal of the filtering circuit; a second end of the second switch tube is connected to the high-voltage ground; a second end of the third switch tube is connected to the high-voltage negative electrode; and a control terminal of the first switch tube, a control terminal of the second switch tube, and a control terminal of the third switch tube are respectively connected to the control circuit.

According to an embodiment of the present application, when the first switch tube is in a conducting state, the first transmitting circuit outputs a positive high voltage of AC square wave; when the second switch tube is in a conducting state, the first transmitting circuit outputs a zero voltage of AC square wave; when the third switch tube is in a conductive state, the first transmitting circuit outputs a negative high voltage of AC square wave.

According to an embodiment of the present application, the first switch tube to the third switch tube have the same structure, and are composed of multiple reversely connected switch tubes.

According to an embodiment of the present application, the first transmitting circuit further comprises: a first resistor and a second resistor, wherein the first resistor is connected in series between a first end of the first switch tube and the high-voltage positive electrode, and the second resistor is connected in series between a second end of the third switch tube and the high-voltage negative electrode.

According to an embodiment of the present application, the filtering circuit comprises: a first inductor and a first capacitor, wherein one end of the first inductor is connected to the transmitting terminal of the first transmitting circuit, the other end of the first inductor is connected to one end of the first capacitor and the first electrode, and the other end of the first capacitor is connected to the high-voltage ground.

According to an embodiment of the present application, the first electrodes comprise multiple electrodes, and each of the first electrodes is provided with the first transmitting circuit and the filtering circuit, and the control circuit is also used to control any one of the first transmitting circuits to form a sine wave between the corresponding first electrode and the second electrode.

According to an embodiment of the present application, either the first electrodes or the second electrodes comprise multiple electrodes, and the high-voltage transmitting circuit further comprises: a first switching circuit and a second switching circuit, and wherein the first transmitting circuit is connected to multiple first electrodes through the first switching circuit, the high-voltage ground is connected to multiple second electrodes through the second switching circuit, and control circuit is also used to control the first switching circuit and the second switching circuit so as to form the sine wave between any one of the first electrodes and any one of the second electrodes.

According to an embodiment of the present application, the first switching circuit comprises multiple first switches which respectively correspond to multiple first electrodes one by one, and a first switch is connected in series between an output terminal of the filtering circuit and a corresponding first electrode; and the second switching circuit comprises multiple second switches which respectively correspond to multiple second electrodes one by one, and a second switch is connected in series between a corresponding second electrode and the high-voltage ground.

According to an embodiment of the present application, the high-voltage transmitting circuit further comprises: a sampling resistor, which is connected in series between a second end of the first transmitting circuit and the high-voltage ground.

According to an embodiment of the present application, the high-voltage transmitting circuit further comprises an overcurrent protection module comprising: a sensing resistor, which is provided at the transmitting terminal of the first transmitting circuit; a transformer, whose primary winding is connected in parallel with the induction resistor; a third resistor, which is connected in parallel with the secondary winding of the transformer, and one end of the third resistor is connected to ground; a fourth resistor, whose one end is connected to the other end of the third resistor; an operational amplifier, whose coaxial input terminal is connected to the other end of the fourth resistor, wherein a positive source voltage terminal of the operational amplifier is connected to a power supply, and a negative source voltage terminal of the operational amplifier is connected to ground; and a fifth resistor, which is connected between the inverting input terminal and the output terminal of the operational amplifier; wherein the output terminal of the operational amplifier, a full-wave peak detection circuit, a filter voltage dividing circuit, an analog-to-digital converter, and a digital chip are connected in sequence.

In order to achieve the above objects, a second embodiment of the present application provides an ablation tool, which comprises a high-voltage transmitting circuit for a catheter as in the above first embodiment.

The ablation tool according to the embodiment of the present application realizes the output of pulsed voltage waveform of a sine wave through the above high-voltage transmitting circuit for the catheter, thereby meeting the treatment needs in some pulsed electric field ablation surgeries, and improving the application prospects of pulsed electric field ablation technology in the treatment of arrhythmia.

Additional aspects and advantages of the application will be set forth in part in the description which follows, and they will be obvious from the description, or may be learned by practice of the application.

### BRIEF DESCRIPTION OF THE DRAWINGS

Fig. 1 is a schematic structural diagram of a high-voltage transmitting circuit for a catheter according to the first embodiment of the present application;
Fig. 2 is a schematic structural diagram of a high-voltage transmitting circuit for a catheter according to the second embodiment of the present application;
Fig. 3 is a schematic structural diagram of a high-voltage transmitting circuit for a catheter according to the third embodiment of the present application;
Fig. 4 is a voltage waveform diagram of a high-voltage transmitting circuit for a catheter according to one embodiment of the present application;
Fig. 5 is a schematic structural diagram of a high-voltage transmitting circuit for a catheter according to the fourth embodiment of the present application;
Fig. 6 is a schematic structural diagram of a high-voltage transmitting circuit for a catheter according to the fifth embodiment of the present application;
Fig. 7 is a schematic structural diagram of a high-voltage transmitting circuit for a catheter according to the sixth embodiment of the present application;
Fig. 8 is a schematic structural diagram of a high-voltage transmitting circuit for a catheter according to the seventh embodiment of the present application;
Fig. 9 is a schematic structural diagram of an overcurrent protection module provided according to the present application;
Fig. 10 is a schematic structural diagram of a full-wave peak detection circuit in the overcurrent protection module;
Fig. 11 is a schematic structural diagram of a filter voltage dividing circuit in the overcurrent protection module;
Fig. 12 is a schematic waveform diagram of the current passing through RSENSE, the voltage at Vo1 terminal and the voltage at Vo2 terminal in the overcurrent protection module;
Fig. 13 is a schematic structural diagram of an ablation tool according to an embodiment of the present application.

### DETAILED DESCRIPTION

Embodiments of the present application are described in detail below, and the examples of which are illustrated in the accompanying drawings, wherein the same or similar reference numerals throughout represent the same or similar elements or elements with the same or similar functions. The embodiments described below with reference to the accompanying drawings are exemplary, and are intended to explain the present application and should not be construed as limiting the present application.

The high-voltage transmitting circuit and ablation tool for a catheter provided by embodiments of the present application will be described below with reference to the accompanying drawings.

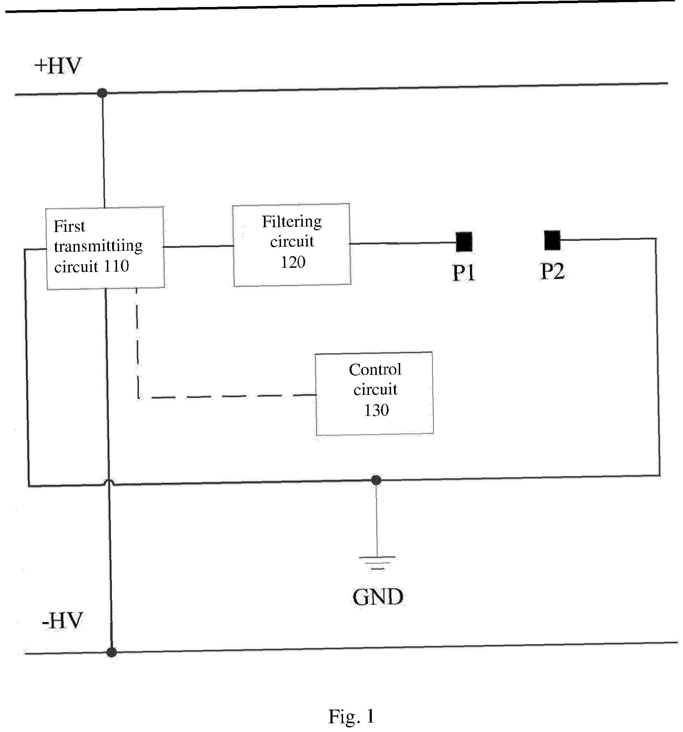

Fig. 1 is a schematic structural diagram of a high-voltage transmitting circuit for a catheter according to the first embodiment of the present application. Referring to Fig. 1, a first electrode P1 and a second electrode P2 are provided on the catheter. The high-voltage transmitting circuit comprises: a first transmitting circuit 110, a filtering circuit 120 and a control circuit 130.

Particularly, the first end of the first transmitting circuit 110 is connected to the high-voltage positive electrode +HV, the second end of the first transmitting circuit 110 is connected to the high-voltage ground GND, and the third end of the first transmitting circuit 110 is connected to the high-voltage negative electrode -HV; the input terminal of the filtering circuit 120 is connected to the transmitting terminal of the first transmitting circuit 110, the output terminal of the filtering circuit 120 is connected to the first electrode P1, and the second electrode P2 is connected to the high-voltage ground GND; the control circuit 130 is connected to the first transmitting circuit 110 for controlling the first transmitting circuit 110 so that the first transmitting circuit 110 outputs an AC square wave, which is filtered by the filtering circuit 120 to form a sine wave between the first electrode P1 and the second electrode P2.

It should be noted that, pulsed electric field ablation technology is a common method for treating arrhythmias. This method generates a high-voltage pulsed electric field with a pulse width of milliseconds, microseconds or even nanoseconds, and releases extremely high levels of energy in a short period of time, so that under the action of high-voltage pulsed electric fields, a large number of irreversible micropores was generated on cell membranes and even intracellular organelles such as endoplasmic reticulum, mitochondria, cell nuclei, etc., thereby causing the apoptosis of diseased cells and thus achieving the purpose of rapidly treating arrhythmia.

In practical applications, electrophysiological examination of the heart is first performed by puncturing the subclavian vein and bilateral femoral veins to clearly diagnose the location of the lesion to be ablated, and then a special catheter is inserted into the heart along the blood vessels and reaches the lesion site, and then high-voltage pulsed electric fields are emitted to the lesion site in a short period of time by the first electrode P1 and the second electrode P2 arranged on the catheter, thereby achieving the function of accurately treating cardiomyocytes without causing unnecessary effects on other non-target cell tissues. During operation, under normal circumstances, the control circuit 130 in the high-voltage transmitting circuit will send a control signal to the first transmitting circuit 110, so as to control the first transmitting circuit 110 to periodically output a positive high voltage, a negative high voltage or a zero voltage, thereby forming an AC square wave pulsed voltage with a certain frequency. The AC square wave pulsed voltage formed by the first transmitting circuit 110 can form a sine wave voltage with a certain frequency at the first electrode P1 under the filtering action of the filtering circuit 120. Since the second electrode P2 is connected to the high-voltage ground GND, the voltage of the second electrode P2 is always zero voltage. Therefore, under the joint action of the control circuit 130 and the filtering circuit 120, a sine wave voltage with a certain frequency may be formed between the first electrode P1 and the second electrode P2; thus the output of pulsed voltage waveform of a sine wave is realized, thereby meeting the treatment needs of some pulsed electric field ablation surgeries. For example, some studies have shown that sine wave pulse ablation can achieve unipolar, bipolar and mixed ablation forms through phase changes between two electrodes, thereby better controlling the depth of ablation damage, and probably the deepest ablation depth is more than 10mm, which greatly exceeds the damage depth of square wave steep pulse ablation. In this way, sine wave pulse ablation may be able to treat more arrhythmia indications, such as ventricular tachycardia and other conditions that require ablation of thicker myocardial tissue. At the same time, due to the inherent characteristics of the sine wave pulse (single frequency), compared with the steep pulse ablation of the square wave, it has less stimulation to the nerve tissue near the ablation site, and the degree of muscle contraction and tremor caused during the ablation operation will also be less, thereby improving patient tolerance during the surgery.

The high-voltage transmitting circuit for a catheter according to an embodiment of the present application, the first transmitting circuit is respectively connected to a filtering circuit and a control circuit. The control circuit is used to control the first transmitting circuit so that the first transmitting circuit outputs an AC square wave, and a filtering process is performed by the filtering circuit to form a sine wave between the first electrode and the second electrode. As a result, the output of pulsed voltage waveform of a sine wave is realized, thereby meeting the treatment needs in some pulsed electric field ablation surgeries and improving the application prospects of pulsed electric field ablation technology in the treatment of arrhythmia.

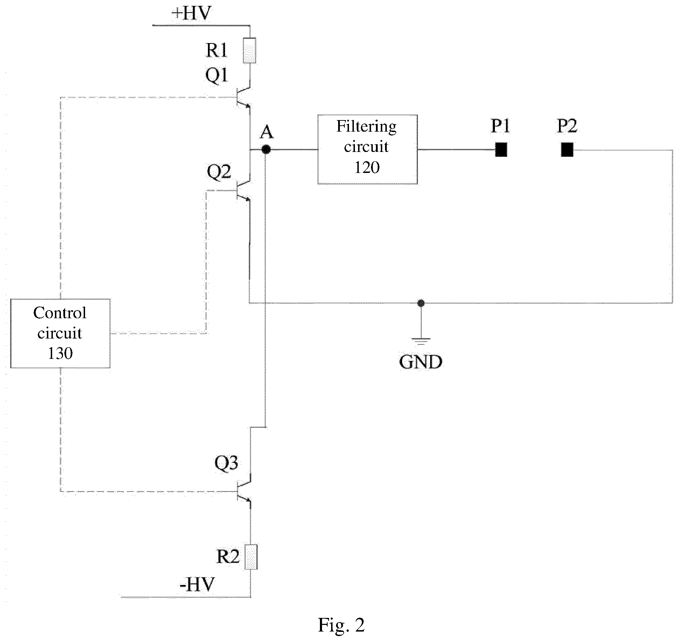

In some embodiments, as shown in Fig. 2, the first transmitting circuit 110 comprises: a first switch tube Q1, a second switch tube Q2 and a third switch tube Q3, wherein a first end of the first switch tube Q1 is connected to the high voltage positive electrode + HV, a second end of the first switch tube Q1, a first end of the second switch tube Q2, and a first end of the third switch tube Q3 are all connected to an input terminal of the filtering circuit 120; and a second end of the second switch tube Q2 is connected to the high-voltage ground GND, a second end of the third switch tube Q3 is connected to the high-voltage negative electrode -HV; and a control terminal of the first switch tube Q1, a control terminal of the second switch tube Q2 and a control terminal of the third switch tube Q3 are respectively connected to the control circuit 130.

Further, when the first switch tube Q1 is in a conducting state, the first transmitting circuit 110 outputs a positive high voltage of AC square wave; when the second switch tube Q2 is in a conducting state, the first transmitting circuit 110 outputs a zero voltage of AC square wave; when the third switch tube Q3 is in a conducting state, the first transmitting circuit 110 outputs a negative high voltage AC square wave.

Particularly, as shown in Fig. 2, the transmitting terminal of the first transmitting circuit 110 is marked as point A, and the control terminals of the first switch tube Q1, the second switch tube Q2 and the third switch tube Q3 in the first transmitting circuit 110 are respectively connected to the control circuit 130. When the control circuit 130 controls the first switch tube Q1 to turn on through the control terminal of the first switch tube Q1, the transmitting terminal A of the first transmitting circuit 110 is directly connected to the high-voltage positive electrode +HV through the first switch tube Q1 to obtain an absolute positive high voltage. When the control circuit 130 controls the second switch tube Q2 to turn on through the control terminal of the second switch tube Q2, the transmitting terminal A of the first transmit circuit 110 is directly connected to high-voltage ground GND through the second switch tube Q2 to obtain an absolute zero voltage. When the control circuit 130 controls the third switch tube Q3 to turn on through the control terminal of the third switch tube Q3, the transmitting terminal A of the first transmit circuit 110 is directly connected to the high-voltage negative electrode -HV through the third switch tube Q3 to obtain an absolute negative high voltage.

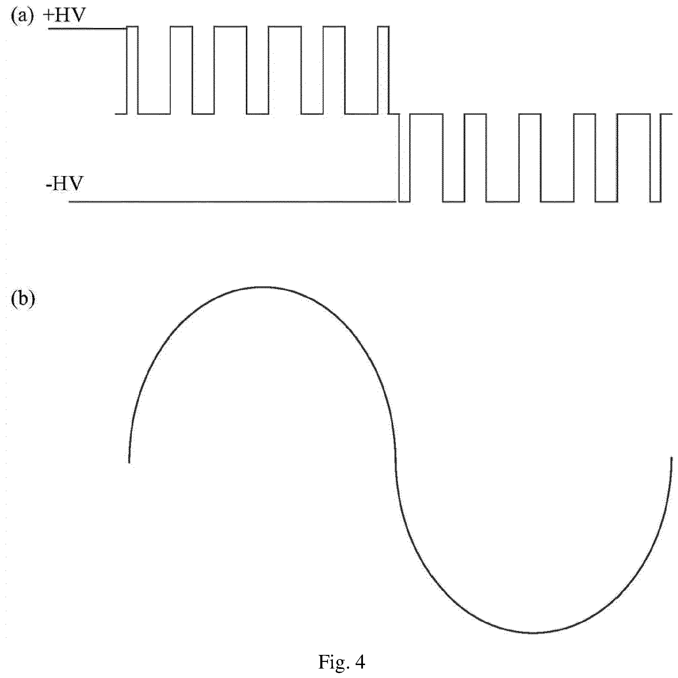

Furthermore, an absolute positive high voltage, zero voltage and negative high voltage may be obtained based on the transmitting terminal A of the first transmitting circuit 110. Therefore, the first switch tube Q1, the second switch tube Q2 and the third switch tube Q3 are controlled to turn on or off according to a certain rule through the control circuit 130, so that a desired pulsed voltage waveform may be obtained at the transmitting terminal A of the first transmitting circuit 110. For example, an AC square wave pulsed voltage waveform as shown in Fig. 4(a) may be obtained.

It should be noted that, the above first switch tube Q1 to third switch tube Q3 are ideal switching models, and in practical application, some or all of the above first switch tube Q1 to third switch tube Q3 may be achieved by one or more non-ideal switch tubes and some auxiliary components to realize the functions of the ideal switching models. All these variations or alternatives are within the protection scope of this application, and they are not specifically limited herein.

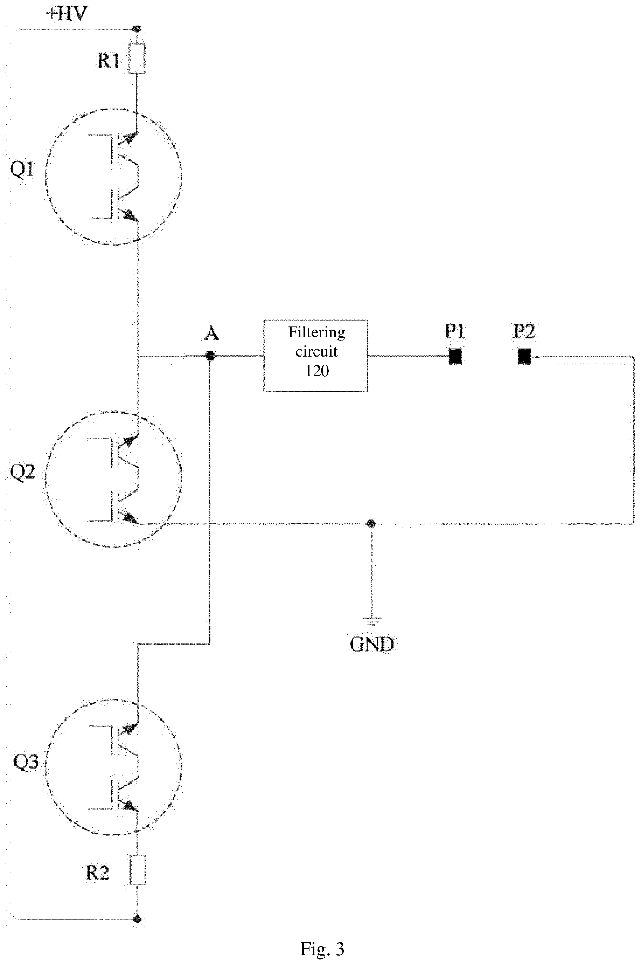

In some embodiments, as shown in FIG. 3, the first switch tube Q1 to third switch tube Q3 have the same structure, and are composed of multiple reversely connected switch tubes. Herein, two reversely connected switch tubes are taken as an example, for the detailed working process please referring to the above description, and it will not be repeated herein.

In some embodiments, continuing to refer to FIG. 2, the first transmitting circuit 110 further comprises: a first resistor R1 and a second resistor R2, wherein the first resistor R1 is connected in series between a first end of the first switch tube Q1 and the high-voltage positive electrode +HV, the second resistor R2 is connected in series between a second end of the third switch tube Q3 and the high-voltage negative electrode -HV

It should be noted that, the first resistor R1 and the second resistor R2 are current-limiting resistors or current shock-proof resistors, which are used to reduce or prevent parasitic inductance or parasitic capacitance from causing instantaneous large current and circuit oscillation when the system is discharging.

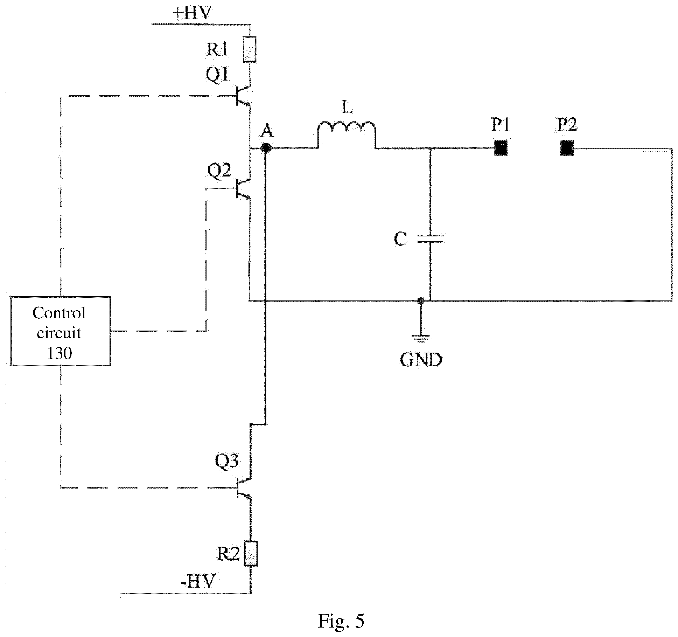

In some embodiments, as shown in FIG. 5, the filtering circuit 120 comprises: a first inductor L and a first capacitor C, wherein one end of the first inductor L is connected to the transmitting terminal of the first transmitting circuit 110, and the other end of the first inductor L is connected to one end of the first capacitor C and the first electrode P1, and the other end of the first capacitor C is connected to the high-voltage ground GND.

Particularly, as shown in FIG. 5, the second electrode P2 is used as the reference electrode, the AC square wave formed by the first transmitting circuit 110 will form pulsed voltage waveform of a sine wave as shown in Fig. 4(b) between the first electrode P1 and the second electrodes P2 under the joint action of the first inductor L and the first capacitor C. It should be noted that, the frequency of the resulting pulsed voltage waveform of a sine wave may be changed by controlling the on/off switching speed of the first switch tube Q1, the second switch tube Q2 and the third switch tube Q3 through the control circuit 130, thereby forming various sine waves with different frequencies to meet the needs of different catheter ablation surgeries.

In addition, when the absolute values of the voltages of the high-voltage positive electrode (+HV) and the high-voltage negative electrode (-HV) are equal, by turning on the second switch tube Q2 to achieve grounding, the transmitting terminal A can obtain absolute zero voltage, so that the sine wave obtained by filtering is centered, making it easier for other generators and other circuits to obtain the sampling value during transmission by referring to the reference end of the ground level, so that the transmitted waveforms between the electrodes are relatively more stable, which is beneficial to the safety of ablation surgery.

When the voltage and phase of the high-voltage positive electrode and/or high-voltage negative electrode are changed, the voltage of the output voltage waveform may be more accurately controlled, thereby giving more waveform options, so that different depths of pulse ablation damage may be achieved to adapt to the thickness of myocardial tissue at different locations in the heart and to treat different indications of arrhythmia.

The high-voltage positive electrode and high-voltage negative electrode of this application may be respectively connected to the total output positive terminal Vout in a technical solution of a patent application filed with China National Intellectual Property Administration on August 10, 2021 by the applicant (application number: CN202110912538.X with a title of "High-voltage Generating Circuit and Ablation Tool for Catheter"; or, application number: CN202110912278.6 with a title of "High-voltage Generating Circuit and Ablation Tool for Catheter), so as to provide different voltages.

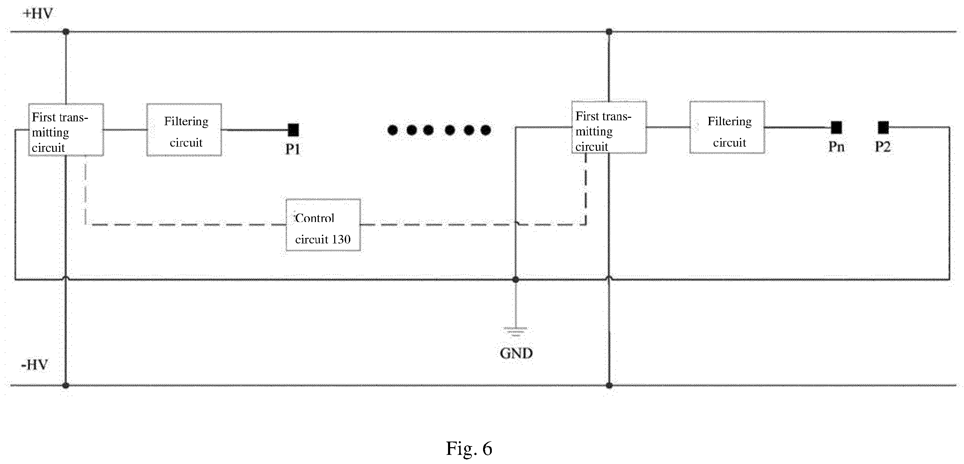

Optionally, as shown in Fig. 6, the first electrodes comprise multiple electrodes, and each of the first electrodes is provided with a first transmitting circuit and a filtering circuit, and a control circuit 130 is also used to control any one of the first transmitting circuits to form a sine wave between the corresponding first electrode and second electrode P2.

That is to say, the high-voltage transmitting circuit in this application may be composed of multiple first transmitting circuits connected in parallel, and respectively output sine waves to multiple first electrodes through filtering circuit, so that desired pulsed voltage waveform of a sine wave is formed between multiple first electrodes and the second electrodes P2. In the course of application, the second electrode P2 is selected as the reference electrode, and the control circuit 130 can control the operation of any one of the first transmitting circuits, so that a sine wave may be formed at any one of the first electrodes, and thus desired pulsed voltage waveform of a sine wave is formed between any one of the first electrodes and the second electrodes P2; that is to say, the output of pulsed voltage waveform of a sine wave may be realized by a combination of any one of the first electrodes and the second electrodes P2, thereby improving the flexibility of voltage regulation between any one of the first electrodes and the second electrodes P2. In addition, since multiple first electrodes may be arranged at different positions of the catheter, during the pulsed voltage ablation treatment process, the working electrode may be adjusted at will according to the location of the lesion, without the need of moving the catheter in a large range to align the electrode with the lesion, thereby improving the operability of pulsed voltage ablation technology and reducing the difficulty of operation.

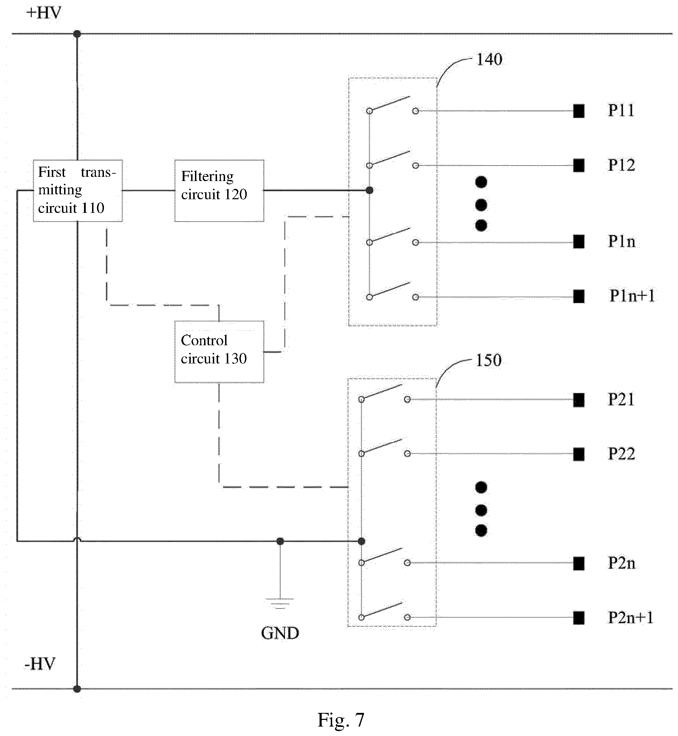

Optionally, as shown in Fig. 7, either the first electrodes or the second electrodes comprise multiple electrodes, and the high-voltage transmitting circuit also comprises: a first switching circuit 140 and a second switching circuit 150, wherein the first transmitting circuit 110 is connected to multiple first electrodes through the first switching circuit 140, the high-voltage ground GND is connected to multiple second electrodes through the second switching circuit 150, and a control circuit 130 is also used to control the first switching circuit 140 and the second switching circuit 150 so as to form a sine wave between any one of the first electrodes and any one of the second electrodes.

Further, the first switching circuit 140 comprises multiple first switches which correspond to multiple first electrodes one by one, and a first switch is connected in series between the output terminal of the filtering circuit 120 and the corresponding first electrode; the second switching circuit 150 comprises multiple second switches which correspond to multiple second electrodes one by one, and a second switch is connected in series between the corresponding second electrode and the high-voltage ground GND.

Particularly, during operation, under normal circumstances, the control circuit 130 in the high-voltage transmitting circuit will respectively send control signals to the first transmitting circuit 110, the first switching circuit 140 and the second switching circuit 150 to control: the connection of any one or more electrodes of the multiple first electrodes to the first transmitting circuit 110 through the filtering circuit 120, and the connection of any one or more electrodes of the multiple second electrodes to the high-voltage ground GND. For example, the control circuit 130 can control the first electrode P11 to be connected to the first transmitting circuit 110 through the filtering circuit 120, and the second electrode P21 to be connected to the high-voltage ground GND, so that pulsed voltage waveform of a sine wave is formed between the first electrode P11 and the second electrode P21; alternatively, the control circuit 130 can control: the connection of the first electrode P11 to the first transmitting circuit 110 through the filtering circuit 120, and the connection of the second electrode P22 to the high-voltage ground GND, so that pulsed voltage waveform of a sine wave is formed between the first electrode P11 and the second electrode P22; by analogy, a sine wave voltage may be formed between the first electrode P11 and any one of the second electrodes; in addition, the control circuit 130 can also control any one of the first electrodes to turn on or off, so that pulsed voltage waveform of a sine wave may be formed between any one of the first electrodes and the second electrodes, thereby realizing the output of the pulsed voltage waveform between any two transmitting electrodes, and improving the flexibility of voltage regulation between any two transmitting electrodes. In addition, since the electrodes may be arranged at different positions on the catheter, during pulsed voltage ablation treatment, the working electrode may be adjusted at will according to the location of the lesion, without the need of moving the catheter in a large range to align the electrode with the lesion, thereby improving the operability of the pulsed voltage ablation technology, reducing the difficulty of operation to better meet the treatment needs of various pulsed electric field ablation surgeries.

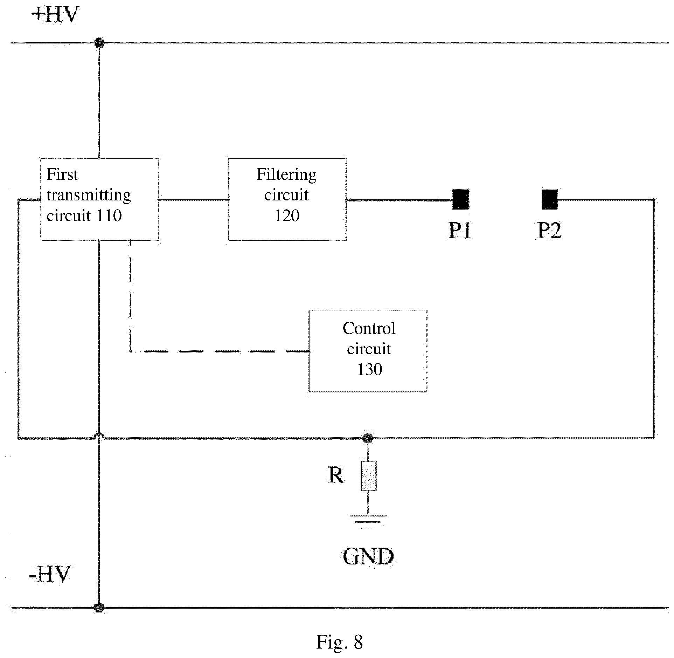

In some embodiments, as shown in FIG. 8, the high-voltage transmitting circuit also comprises: a sampling resistor R, which is connected in series between a second end of the first transmitting circuit 110 and the high-voltage ground GND for detecting high voltage discharge during use.

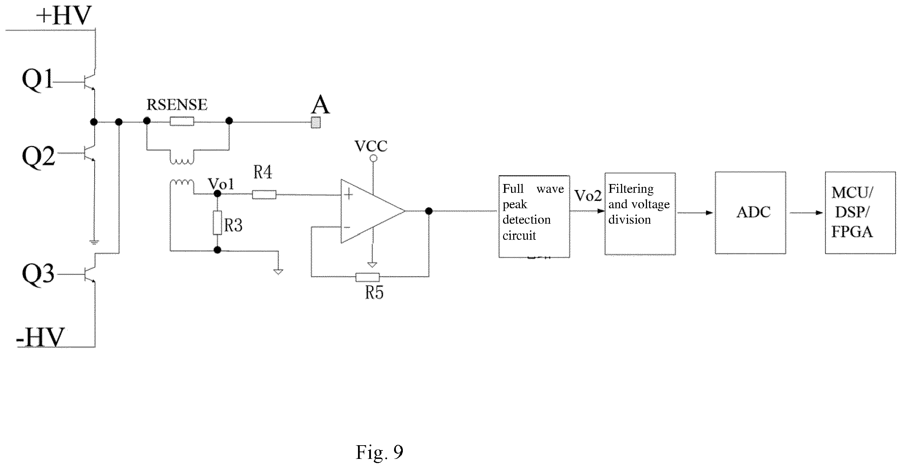

In some embodiments, as shown in Fig. 9, the above high-voltage transmitting circuit also comprises an over-current protection module comprising: a sensing resistor (RSENSE), which is provided at the transmitting terminal of the first transmitting circuit; a transformer, whose primary winding is connected in parallel with the induction resistor; a third resistor R3, which is connected in parallel with the secondary winding of the transformer, and one end of the third resistor R3 is connected to ground; a fourth resistor R4, whose one end is connected to the other end of the third resistor R3; an operational amplifier, whose coaxial input terminal is connected to the other end of the fourth resistor R4, wherein a positive source voltage terminal of the operational amplifier is connected to a power supply, and a negative source voltage terminal of the operational amplifier is connected to ground; and a fifth resistor R5, which is connected between the inverting input terminal and the output terminal of the operational amplifier for mainly functioning to match the in-phase terminal impedance; wherein the output terminal of the operational amplifier, a full-wave peak detection circuit, a filter voltage dividing circuit, an analog-to-digital converter (ADC), and a digital chip (such as MCU/DSP/FPGA) are connected in sequence.

It should be noted that, in Figs. 9-11, R3-R15 respectively represent the third to fifteenth resistors, C1 and C2 respectively represent a first capacitor and a second capacitor, and D1 and D2 respectively represent a first diode and a second diode; the triangle represents an operational amplifier, and VCC represents the power supply voltage.

The above overcurrent protection module detects the voltage change at both ends of a sensing resistor (RSENSE) through a transformer, and the operational amplifier follows. The full-wave peak detection circuit performs voltage flipping and peak detection. After filtering and voltage division, and ADC sampling, a real-time tracking function for the current of power circuit (A-terminal output current) is realized through digital chip such as FPGA/DSP/MCU. When the detected current is greater than a preset value, the digital chip (such as MCU/DSP/FPGA) controls to quickly disconnect switches Q1 and Q3 to prevent harm to a patient, thereby ensuring the ablation surgeries and other procedures to be performed more safely.

It should be noted that, a third resistor R3 provided in the overcurrent protection module can quickly release the energy of the secondary winding of a transformer, so that the voltage at Vo1 can sensitively respond to the current passing through RSENSE, thereby facilitating the subsequent detection results of digital chip such as MCU/DSP/FPGA to reflect the current at terminal A in real time. In addition, an operational amplifier provided in the overcurrent protection module as a follower may prevent the Vo1 voltage from being low and affecting subsequent detection results.

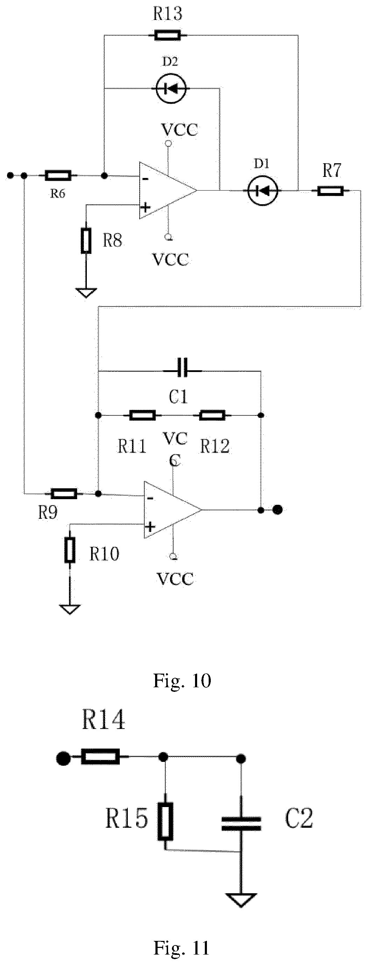

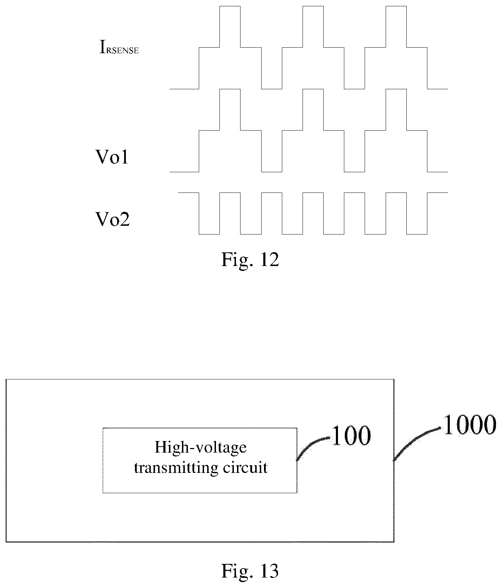

As shown in Fig. 12, the voltage at Vo1 terminal has positive and negative voltages. The full-wave peak detection circuit in this embodiment can perform voltage inversion (converting to positive voltage) of the voltage at Vo1 terminal to facilitate ADC sampling. Particularly, the components in the full-wave peak detection circuit and their connection modes are shown in Fig. 10, for example, the left position of R6 of the full-wave peak detection circuit is connected to an output terminal of the operational amplifier as an input terminal, and other connection modes will not be described in detail herein. Particularly, the current voltage drop of a diode is generally 0.5-0.7V. However, in the present application, the voltage drop of diode D2 is 0.1-0.2V, preferably, the voltage drops of diodes D1 and D2 are both 0.1-0.2V, so that as shown in Fig. 12, the voltage at the Vo2 terminal may better reflect the voltage at the Vo1 terminal, thereby making the detection result more accurate.

The components in the filter voltage dividing circuit in this embodiment and their connection modes are shown in Fig. 11. Particularly, the capacitor C7 is preferably less than or equal to 1nF, so that the voltage output by the filter voltage dividing circuit can reflect the voltage peak (instantaneous peak) in real time, so that the detection results obtained by ADC sampling and digital chips (MCU/DSP/FPGA, etc.) can reflect the real-time A-terminal output current, so as to obtain a more accurate current applied to a patient during ablation surgery.

To sum up, a first transmitting circuit is respectively connected to a filtering circuit and a control circuit, wherein the control circuit is used to control the first transmitting circuit so that the first transmitting circuit outputs an AC square wave, and performs filtering processing through the filtering circuit to form a sine wave between a first electrode and a second electrode, which may be used to treat more arrhythmia indications (such as ventricular tachycardia and other conditions that require ablation at thicker myocardial tissue), causing smaller stimulation to the nerve tissues near the ablation site, reducing the degree of muscle contraction and tremor caused during ablation surgery, enhancing a patient's tolerance during the surgery, and thereby improving the application prospects of pulsed electric field ablation technology in the treatment of arrhythmia. The arrangement of an overcurrent protection module can prevent excessive current from causing harm to a patient, ensuring the ablation surgeries and other procedures to be performed more safely.

FIG. 13 is a schematic structural diagram of an ablation tool according to an embodiment of the present application. Referring to FIG. 13, the ablation tool 1000 comprises the above high-voltage transmitting circuit 100 for a catheter.

The ablation tool according to the embodiment of the present application realizes the output of pulsed voltage waveform of a sine wave through the above high-voltage transmitting circuit for the catheter, thereby meeting the treatment needs in some pulsed electric field ablation surgeries, and improving the application prospects of pulsed electric field ablation technology in arrhythmia treatment.

It should be noted that, the logic and/or steps represented in the flowcharts or otherwise described herein, for example, may be considered to be a fixed order of executable instructions for implementing logical functions, which may be specifically embodied in any computer-readable medium for use by, or in conjunction with, an instruction execution system, apparatus, or device (such as a computer-based system, a system comprising a processor, or other system that can retrieve and execute instructions from such instruction execution system, apparatus, or device). For the purposes of this specification, a "computer-readable medium" may be any device that can contain, store, communicate, transmit, or transfer a program for use by or in connection with an instruction execution system, apparatus, or device. More specific examples (non-exhaustive list) of computer readable media comprise the following: an electrical connection with one or more wires (electronic device), a portable computer disk cartridge (magnetic device), a random access memory (RAM), a read-only memory (ROM), an erasable and programmable read-only memory (EPROM or flash memory), a fiber optic device, and a portable compact disc read-only memory (CDROM). Additionally, the computer-readable media may even be paper or other suitable media on which the program may be printed, as the paper or other medium for example, may be optically scanned, and subsequently edited, interpreted, or processed with other suitable manner as necessary to obtain the program electronically, and then store it in computer memory.

It should be understood that various parts of the present application may be implemented by hardware, software, firmware, or a combination thereof. In the above embodiments, various steps or methods may be implemented by software or firmware stored in a memory and executed by a suitable instruction execution system. For example, if it is implemented by hardware, as in another embodiment, it may be implemented by any one or a combination of the following technologies known in the art: discrete logic circuit of logic gate circuit for implementing a logic function on a data signal, application-specific integrated circuit with suitable combinational logic gate circuit, programmable gate array (PGA), field programmable gate array (FPGA), MCU, DSP, etc.

In the description of this specification, reference terms "one embodiment," "some embodiments," "an example," "a specific example," or "some examples" or the likes mean that a specific feature, structure, material or characteristic described in conjunction with such an embodiment or example is encompassed in at least one embodiment or example of the application. In this specification, schematic representations of the above terms do not necessarily refer to the same embodiments or examples. Furthermore, the specific feature, structure, material or characteristic described may be combined in any suitable manner in any one or more embodiments or examples.

In addition, the terms "a first" and "a second" are used for descriptive purposes only, and cannot be understood as indicating or implying relative importance, or implicitly indicating quantity of the indicated technical features. Therefore, a feature defined as "a first" or "a second" may explicitly or implicitly comprise at least one of these features. In the description of the present application, "multiple" means at least two, such as two, three, etc., unless otherwise expressly and specifically defined.

In the present application, unless otherwise clearly stated and defined, the terms "installation", "connection", "connected", "fixed" and other terms should be understood in a broad sense. For example, it may be a fixed connection or a detachable connection, or integrated into one; it may be a mechanical connection or an electrical connection; it may be a direct connection or an indirect connection through an intermediate medium; it may be an internal connection between two elements or an interactive relationship between two elements, unless otherwise clearly specified. For those of ordinary skilled in the art, the specific meanings of the above terms in the present application may be understood according to specific circumstances.

Although the embodiments of the present application have been shown and described above, it may be understood that the above embodiments are illustrative, and should not be construed as limitations of the present application. Those of ordinary skilled in the art can make transformation, modifications, substitutions or variations to the above embodiments within the scope of the present application.

## Claims

1. A high-voltage transmitting circuit for a catheter, wherein a first electrode and a second electrode are provided on the catheter, and the high-voltage transmitting circuit comprises:
a first transmitting circuit, wherein a first end of the first transmitting circuit is connected to the high-voltage positive electrode, a second end of the first transmitting circuit is connected to the high-voltage ground, and a third end of the first transmitting circuit is connected to the high-voltage negative electrode;
a filtering circuit, wherein an input terminal of the filtering circuit is connected to a transmitting terminal of the first transmit circuit, an output terminal of the filtering circuit is connected to the first electrode, and the second electrode is connected to the high-voltage ground;
a control circuit, wherein the control circuit is connected to the first transmitting circuit, and is used to control the first transmitting circuit so that the first transmitting circuit outputs an alternating-current (AC) square wave, which is subjected to filter processing by the filtering circuit to form a sine wave between the first electrode and the second electrode.

2. The high-voltage transmitting circuit for a catheter according to claim 1, wherein the first transmitting circuit comprises: a first switch tube, a second switch tube and a third switch tube; and wherein a first end of the first switch tube is connected to the high-voltage positive electrode; a second end of the first switch tube, a first end of the second switch tube, and a first end of the third switch tube are all connected to an input terminal of the filtering circuit; a second end of the second switch tube is connected to the high-voltage ground; a second end of the third switch tube is connected to the high-voltage negative electrode; and a control terminal of the first switch tube, a control terminal of the second switch tube, and a control terminal of the third switch tube are respectively connected to the control circuit.

3. The high-voltage transmitting circuit for a catheter according to claim 2, wherein
when the first switch tube is in a conducting state, the first transmitting circuit outputs a positive high voltage of AC square wave;
when the second switch tube is in a conducting state, the first transmitting circuit outputs a zero voltage of AC square wave;
when the third switch tube is in a conductive state, the first transmitting circuit outputs a negative high voltage of AC square wave.

4. The high-voltage transmitting circuit for a catheter according to claim 2, wherein the first switch tubes to the third switch tube have the same structure, and are composed of multiple reversely connected switch tubes.

5. The high-voltage transmitting circuit for a catheter according to claim 2, wherein the first transmitting circuit further comprises: a first resistor and a second resistor, and wherein the first resistor is connected in series between a first end of the first switch tube and the high-voltage positive electrode, and the second resistor is connected in series between a second end of the third switch tube and the high-voltage negative electrode.

6. The high-voltage transmitting circuit for a catheter according to any one of claims 1 to 5, wherein the filtering circuit comprises: a first inductor and a first capacitor, and wherein one end of the first inductor is connected to the transmitting terminal of the first transmitting circuit, the other end of the first inductor is connected to one end of the first capacitor and the first electrode, and the other end of the first capacitor is connected to the high-voltage ground.

7. The high-voltage transmitting circuit for a catheter according to any one of claims 1 to 5, wherein the first electrodes comprise multiple electrodes, and each of the first electrodes is provided with a first transmitting circuit and a filtering circuit, and a control circuit is also used to control any one of the first transmitting circuits to form a sine wave between the corresponding first electrode and the second electrode.

8. The high-voltage transmitting circuit for a catheter according to any one of claims 1 to 5, wherein either the first electrodes or the second electrodes comprise multiple electrodes, and the high-voltage transmitting circuit further comprises: a first switching circuit and a second switching circuit, and wherein the first transmitting circuit is connected to multiple first electrodes through the first switching circuit, the high-voltage ground is connected to multiple second electrodes through the second switching circuit, and a control circuit is also used to control the first switching circuit and the second switching circuit so as to form a sine wave between any one of the first electrodes and any one of the second electrodes.

9. The high-voltage transmitting circuit for a catheter according to claim 8, wherein the first switching circuit comprises multiple first switches which respectively correspond to multiple first electrodes one by one, and a first switch is connected in series between an output terminal of the filtering circuit and a corresponding first electrode;
the second switching circuit comprises multiple second switches which respectively correspond to multiple second electrodes one by one, and a second switch is connected in series between a corresponding second electrode and the high-voltage ground.

10. The high-voltage transmitting circuit for a catheter according to claim 1, wherein the high-voltage transmitting circuit further comprises: a sampling resistor, which is connected in series between a second end of the first transmitting circuit and the high-voltage ground.

11. The high-voltage transmitting circuit for a catheter according to any one of claims 1 to 10, wherein the high-voltage transmitting circuit further comprises an overcurrent protection module comprising:
a sensing resistor, which is provided at the transmitting terminal of the first transmitting circuit;
a transformer, whose primary winding is connected in parallel with the induction resistor;
a third resistor, which is connected in parallel with the secondary winding of the transformer, and one end of the third resistor is connected to ground;
a fourth resistor, whose one end is connected to the other end of the third resistor;
an operational amplifier, whose coaxial input terminal is connected to the other end of the fourth resistor, wherein a positive source voltage terminal of the operational amplifier is connected to a power supply, and a negative source voltage terminal of the operational amplifier is connected to ground; and
a fifth resistor, which is connected between the inverting input terminal and the output terminal of the operational amplifier;
wherein the output terminal of the operational amplifier, a full-wave peak detection circuit, a filter voltage dividing circuit, an analog-to-digital converter, and a digital chip are connected in sequence.

12. An ablation tool, wherein it comprises the high-voltage transmitting circuit for a catheter according to any one of claims 1 to 11.
